# EUROPÄISCHE PATENTSCHRIFT

(11) **EP 1 656 214 B1**
(45) Veröffentlichungstag und Bekanntmachung des Hinweises auf die Patenterteilung: **24.02.2010**
(21) Anmeldenummer: 04738153.8
(22) Anmeldetag: 18.08.2004
(51) Int. Cl.: B05C 17/005, A61F 2/46, A61B 17/00, B65D 81/32, A61J 1/00

(54) **ANORDNUNG UND VERFAHREN ZUM TRANSFER, MISCHEN UND AUSTRAGEN VON KOMPONENTEN**
DEVICE AND METHOD FOR TRANSFERRING, MIXING AND DELIVERING COMPONENTS
DISPOSITIF ET PROCEDE POUR TRANSFERER, MELANGER ET DISTRIBUER DES COMPOSANTS

(30) Priorität: 21.08.2003 CH 142403
(43) Veröffentlichungstag der Anmeldung: 17.05.2006
(73) Patentinhaber: Medmix Systems AG, 6343 Rotkreuz (CH)
(72) Erfinder: KELLER, Wilhelm, A., CH-6402 Merlischachen (CH)
(74) Vertreter: AMMANN PATENTANWÄLTE AG BERN
(86) Internationale Anmeldenummer: PCT/CH2004/000518
(87) Internationale Veröffentlichungsnummer: WO 2005/018831

(56) Entgegenhaltungen:
- EP-A- 0 327 519
- DE-C- 632 579
- US-A- 6 113 571
- US-A1- 2003 012 747

## Beschreibung

Die vorliegende Erfindung bezieht sich auf eine Anordnung und Verfahren zum Transfer, Mischen und Austragen von Komponenten, mit mindestens zwei Austragvorrichtungen gemäss Oberbegriff der unabhängigen Patentansprüche 1 und 9.

Eine solchen Anordnung ist aus der EP-0 292 472 bekannt, die ein Set zur Bereitstellung und Applikation eines Gewebeklebstoffes offenbart, wobei jeweils vier Spritzenkörper paarweise über ein Kupplungsstück zu einer Einheit vereinigt sind. Das Kupplungsstück weist je einen Konus zur Aufnahme eines entsprechenden konischen Teils an der Spritze auf, wobei eine solche Konus-Verbindung bei höheren Drücken und bei Anwendungen mit hohen Anforderungen an die Sicherheit nicht genügt. Ausserdem wird jeweils nur eine Verbindung zwischen zwei gegenüberliegenden oder nebeneinanderliegenden Spritzen offenbart.

Bei gewissen Anwendungen ist es notwendig, bis zu vier oder aber auch mehr Komponenten miteinander zu vermischen und auszutragen. Dabei können die Komponenten sowohl flüssig als auch pulver- oder granulatförmig oder porös sein. In der Regel werden die Komponenten getrennt gelagert und durch Schütteln, Rühren und/oder mit einem statischen Mischer gemischt, oder aufgelöst.

Es ist davon ausgehend Aufgabe der vorliegenden Erfindung eine oben definierte Anordnung und ein Verfahren anzugeben, die nebst einfacher Bedienung und gutes Vermischen eine hohe Betriebssicherheit gewährleistet. Eine Anordnung und ein Verfahren, die diese Aufgabe lösen, sind in den Patentansprüchen 1 und 9 beschrieben.

Die Erfindung wird im Folgenden anhand von Zeichnungen von Ausführungsbeispielen näher erläutert.
- Fig. 1: zeigt in perspektivischer Sicht ein erstes Ausführungsbeispiel einer erfindungsgemässen Anordnung mit parallel zueinander angeordneten Spritzen,
- Fig. 2: zeigt in einer Sicht von vorne und teilweise geschnitten die erste Doppel-Austragvorrichtung von Fig. 1, mit zusätzlichen Mischeinrichtungen,
- Fig. 3: zeigt in einer Seitenansicht und teilweise geschnitten die andere Doppel-Austragvorrichtung von Fig. 1,
- Fig. 4: zeigt einen Schnitt gemäss der Linie IV-IV von Figur 1;
- Fig. 5: zeigt in einer Sicht von vorne und teilweise geschnitten die erste Doppel-Austragvorrichtung von Fig. 1 mit aufgesetztem Mischer,
- Fig. 6: zeigt eine Ausführungsvariante mit zwei frontal gegeneinander verbundenen Doppelspritzen,
- Fig. 7: zeigt einen Schnitt gemäss Linie VII-VII in Fig. 6,
- Fig. 8: zeigt eine Variante zum ersten Beispiel gemäss Figur 1, und
- Fig. 9: zeigt einen Schnitt gemäss Linie IX-IX in Fig. 8,

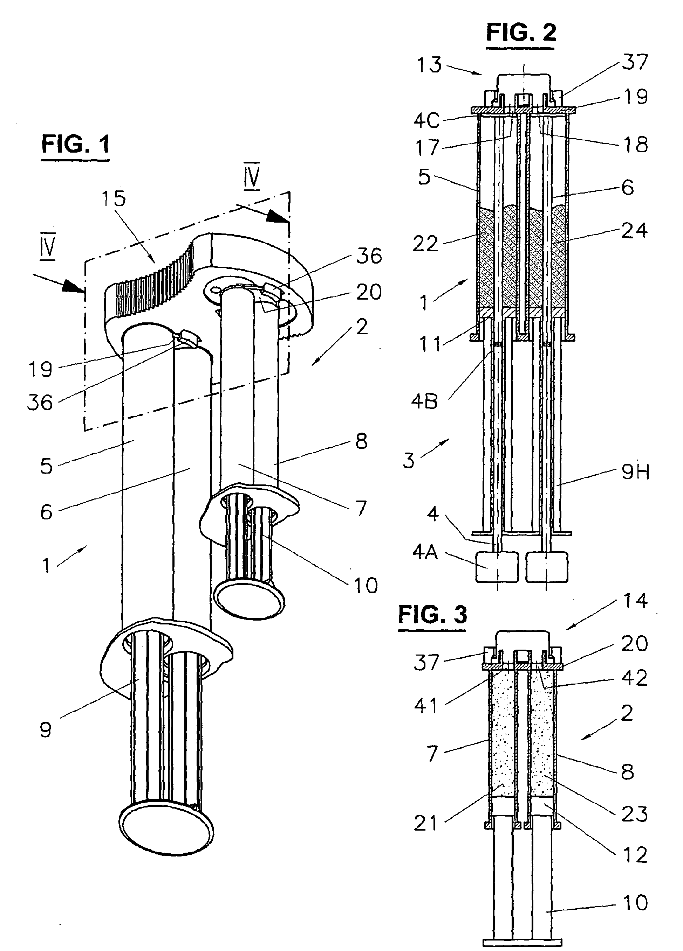

Das erste Ausführungsbeispiel einer erfindungsgemässen Anordnung enthält eine erste und zweite Doppel-Austragvorrichtung, im Folgenden Doppelspritze 1 und Doppelspritze 2 genannt, mit je zwei Vorratsbehältern 5 und 6, bzw. 7 und 8. Jede Doppelspritze weist einen Doppelstössel 9 und 10 auf, an denen Kolben 11 und 12 angeordnet sind, s. Figuren 2 bis 5 und ist mit einer Verschlusskappe 13 und 14 verschlossen, s. Figuren 2 und 3.

Die beschriebenen Doppelspritzen sind auf dem Markt erhältlich. Das Wesentliche für die erfindungsgemässe Anordnung ist der Transfer des Inhalts der einen Doppelspritze in den Inhalt der anderen Doppelspritze und das anschliessende Austragen über einen Mischer oder ein Zubehörteil wie Spitze oder dergleichen oder der Transfer der Komponenten der Doppelspritzen zu einem gemeinsamen Anschluss für einen Mischer oder ein Zubehörteil. Die Verbindung zwischen den beiden Doppelspritzen wird durch die Transfereinheit gewährleistet, die je nach Ausführungsform die oben beschriebenen Transferarten realisiert.

Anstatt Doppelspritzen können auch übliche Doppelkartuschen verwendet werden, deren Kolben durch die Stössel eines Austraggerätes angetrieben werden. Ausserdem gelten alle Beispiele für die Doppel-Austragvorrichtungen sinngemäss für Einfach-, oder Mehrfach- Austragvorrichtungen.

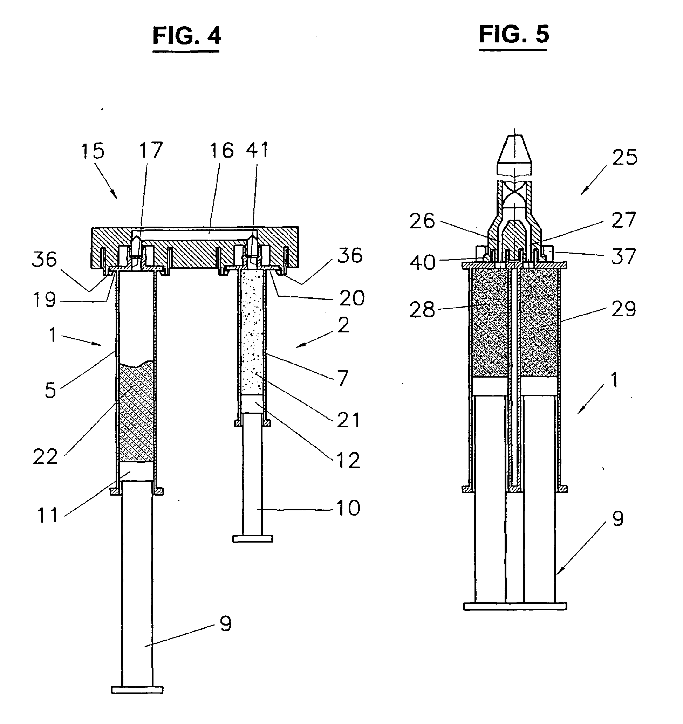

In der ersten Ausführungsvariante weist die Transfereinheit 15 zwei Transferkanäle auf, wobei in Fig. 4 nur der vordere Transferkanal 16 sichtbar ist, der die Verbindung zwischen den vorderen zwei Auslässen 17 und 41 der Vorratsbehälter 5 und 7 der Doppelspritzen 1 und 2 herstellt.

Wie aus den Figuren 1 und 4 hervorgeht, sind die Spritzen mit der Transfereinheit über je eine lösbare Verriegelung verbunden, wobei die Verriegelungselemente 36 an der Transfereinheit den Flansch 19, bzw. 20 an den Spritzen 1 und 2 hintergreifen.

In den Figuren 2 und 3 sind die beiden Doppelspritzen 1 und 2 in gefülltem Zustand, wie in Fig. 1 dargestellt, wobei beide Spritzen je eine Verschlusskappe 13 bzw. 14 aufweisen.

Bei der Ausführungsvariante gemäss den Figuren 1 bis 5 wird beim Befestigen der beiden Doppelspritzen an der Transfereinheit eine Verbindung zwischen den Auslässen der Doppelspritze hergestellt. Damit kann z.B. die Flüssigkeit der Vorratsbehälter 7 und 8 von Doppelspritze 2 in die Vorratsbehälter 5 und 6 der Doppelspritze 1 gelangen, um dort mit den sich darin befindlichen Komponenten vermischt zu werden. Dabei gelangt die flüssige Komponente 21 aus Vorratsbehälter 7 zur Komponente 22 in Vorratsbehälter 5, wobei diese ein Pulver sein kann und die flüssige Komponente 23 aus Vorratsbehälter 8 zur Komponente 24 in Vorratsbehälter 6, die auch ein Pulver oder Granulat sein kann.

Nach dem Transfer mittels des Doppelstössels 10 werden die Komponenten durch Schütteln oder Rühren vermischt oder aufgelöst und die Doppelspritze 2 kann entfernt und entsorgt werden. Die Transfereinheit 15 kann ebenfalls entfernt werden und die mit den Gemischen 28 und 29 gefüllte Spritze 1 kann über den Bajonettanschluss 37 mit einem Mischer 25, s. Fig. 5, oder mit einem anderen Zubehör versehen werden.

Die Behälter 5 und 6 der Doppelspritze 1, von der das Gemisch ausgetragen wird, enthalten gemäss Figur 2 je eine Mischeinrichtung 3, die einen Mischstab 4 mit Drehknopf 4A, Sollbruchstelle 4B zum Abbrechen nach dem Mischen und Mischscheibe 4C, die beispielsweise gelocht und/oder mit am Umfang angeordneten Ausnehmungen versehen sein kann, oder sonstwie gestaltet sein kann. Dabei sind die Stössel als Hohlstössel 9H ausgebildet, in denen der Mischstab geführt ist. Zum Vermischen wird der Stab hin- und herbewegt sowie gedreht. Einerseits braucht nicht jeder Behälter eine Mischeinrichtung aufweisen, andererseits können die Behälter auch mit anderen Mischeinrichtungen ausgestattet sein. Ausserdem wird hier unter Mischen auch das Auflösen einer Komponente in einer anderen verstanden.

Die beiden Gemische 28 und 29, die aus der Mischung von Komponente 21 mit Komponente 22, bzw. Komponente 23 mit Komponente 24 stammen, werden mittels des Doppelstössels 9 durch den Mischer 25 getrieben, wobei der Mischer 25 Einlässe 26 und 27 sowie Mischelemente 30 aufweist.

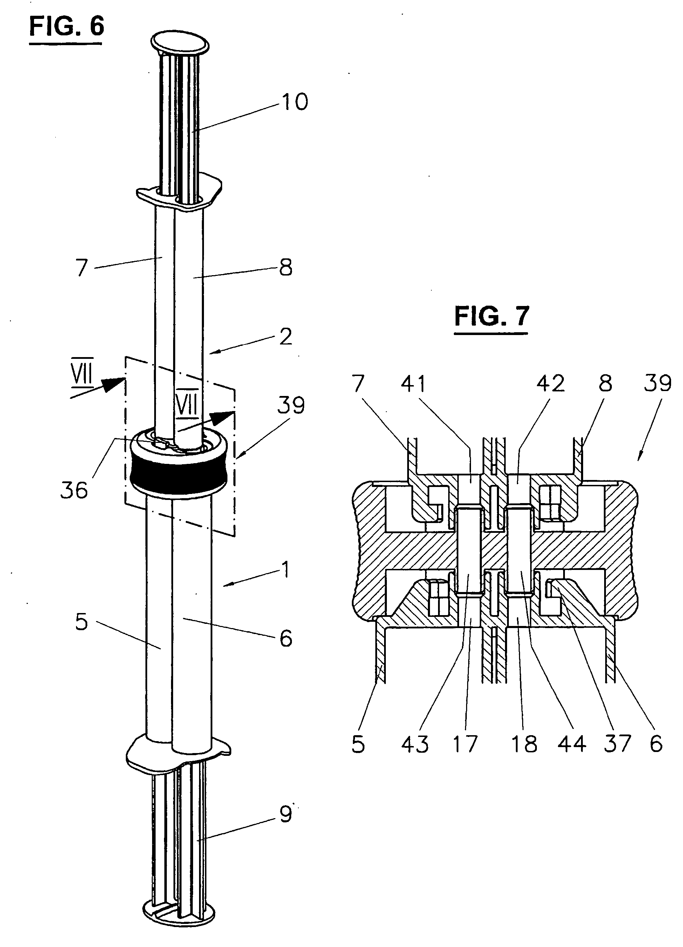

Im Ausführungsbeispiel gemäss den Figuren 6 und 7 sind die Doppelspritzen 1 und 2 nicht parallel zueinander in der Transfereinheit 39 angeordnet, sondern frontal gegeneinander. Auch hier hintergreifen die Verriegelungselemente 36 an der Transfereinheit 39 die Flansche 19, bzw. 20 der Spritzen. Der Schnitt von Fig. 7 stellt die beiden Auslässe 17 und 18 der Spritze 1 dar sowie die Bajonettanschlussteile 37, die dem Anschluss eines Mischers oder Zubehörs dienen. Sinngemäss gilt das Gleiche für die Auslässe 41, 42 und Bajonettanschlussteile 37 von Spritze 2. Die beiden Auslässe 17 und 18 sind über zwei Verbindungskanäle 43 und 44 mit den Auslässen 41 und 42 verbunden.

Die Arbeitsweise ist ähnliche wie beim ersten Ausführungsbeispiel, in dem zuerst die beiden Spritzen auf die Transfereinheit aufgesetzt werden, die Flüssigkeit aus Doppelspritze 2 in die Doppelspritze 1 überführt wird und anschliessend Spritze 2 sowie die Transfereinheit von Spritze 1 abgenommen werden und nach dem Mischen durch Schütteln oder mittels einer Mischeinrichtung 3 auf Spritze 1 ein Mischer oder ein Zubehör aufgesetzt werden kann, wonach die Gemische von Spritze 1 ausgetragen werden. Auch hier wäre es theoretisch möglich, eine Transfereinheit für mehr als zwei Doppelspritzen vorzusehen.

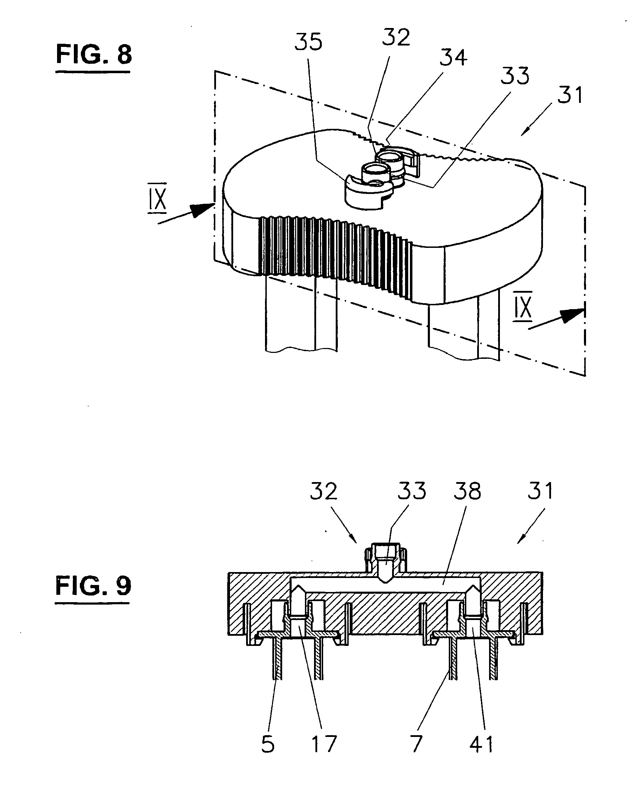

In den Figuren 8 und 9 ist eine Ausführungsvariante dargestellt, bei welcher die Komponenten nicht zuerst von einer Doppelspritze zur anderen und dann zum Mischer transferiert werden, sondern alle vier Komponenten je paarweise zusammengefasst werden und zu einem gemeinsamen Anschluss gelangen. Die Transfereinheit 31 weist einen Anschluss 32 mit zwei Auslässen 33 und 34 sowie Bajonetthalterungen 35 auf, die dem Anschluss eines Mischers oder von anderen Zubehören dienen.

Die Auslässe 17 und 41 sowie die Verbindungsflansche 19 und 20 der Spritzen sind dieselben wie bei der Variante gemäss Fig. 1 und die Transfereinheit weist spritzenseitig dieselben Verriegelungselemente 36 auf, wie sie aus Fig. 1 ersichtlich sind. Die Verbindungskanäle, wovon nur einer, Kanal 38 in Fig. 9, sichtbar ist, verbindet die beiden Auslässe 17 und 41 und mündet in Auslass 33, während der andere, nicht eingezeichnete Verbindungskanal in Auslass 34 mündet. Es ist für gewisse Anwendungszwecke denkbar, dass die beiden Verbindungskanäle in einen gemeinsamen Auslass münden, und nicht in je einen Auslass 33 und 34.

Ausgehend von den oben beschriebenen Ausführungsbeispielen ist es möglich, über eine angepasste Transfereinheit mehr als zwei Doppelspritzen miteinander zu verbinden, wobei dem Gemisch während dem Austragen eine weitere Flüssigkeit, wie Hormone, Antibiotika u. dgl. beigemischt werden kann oder eine zweite Doppelspritze oder auch Einfachspritze mit der zuletzt austragenden Doppelspritze 1 verbunden werden kann.

Zum Austragen kann auf die Doppelspritze anstatt des statischen Mischers ein beliebiges Austrittstück aufgesetzt werden. Das Mischen der Gemische 28 und 29 kann durch Schütteln der Doppelspritze 1 oder durch die Mischeinrichtung 4 erfolgen.

Die Vorratsbehälter der Doppelspritzen können sowohl in der Länge als auch im Durchmesser verschieden gross sein. Die Doppelspritzen müssen nicht, wie eingezeichnet, parallel nebeneinander angeordnet sein. Die austragende Doppelspritze kann auch in ein Austraggerät eingesetzt werden, wie dies bei Doppelkartuschen die Regel ist. Die Komponenten in der austragenden Doppelspritze können pulverförmig oder flüssig sein. Ausserdem müssen die Auslässe 33 und 34 nicht gleichgeartet sein und können verschiedene Durchmesser aufweisen. Sinngemäss gilt das für Doppelspritzen offenbarte auch für Doppelkartuschen, oder für Einfachspritzen oder - kartuschen, oder für andere miteinander verbundene Austragvorrichtungen.

## Patentansprüche

1. Anordnung zum Transfer, Mischen und Austragen von Komponenten, mit mindestens zwei Austragvorrichtungen (1, 2), wobei mindestens eine Austragvorrichtung (2) mindestens eine flüssige Komponente (21, 23) aufweist und die Anordnung eine Transfereinheit (15, 31, 39) zum Anschluss der Austragvorrichtungen enthält, die Verbindungskanäle (16; 38; 43, 44) zwischen zwei Auslässen (17, 18; 41,42) der Komponenten (21, 23; 22, 24) der nebeneinander- oder gegenüberliegenden Austragvorrichtungen (1, 2) aufweist, wobei die mindestens zwei Austragvorrichtungen (1, 2) je mindestens zwei Vorratsbehälter (5 - 8) aufweisen, **dadurch gekennzeichnet, dass** die Vorratsbehälter über einen Flansch (19, 20) miteinander verbunden sind und die Transfereinheit (15, 31, 39) Verriegelungselemente (36) aufweist, die mit entsprechenden Verriegelungselementen (37) an den Flanschen (19, 20) der Austragvorrichtungen (1, 2) hintergreifen.

2. Anordnung nach Anspruch 1, **dadurch gekennzeichnet, dass** die Verbindungskanäle (16) der abnehmbar befestigbaren Transfereinheit (15, 39) ausgebildet sind, die flüssigen Komponenten (21, 23) der einen Austragvorrichtung (2) in eine andere, parallel dazu angeordnete Austragvorrichtung (1) zu überführen.

3. Anordnung nach Anspruch 2, **dadurch gekennzeichnet, dass** die Transfereinheit (15) Verbindungskanäle (16) zwischen je einem Auslass (17, 18; 41, 42) eines Vorratsbehälters (5, 7; 6, 8) der einen und der anderen Austragvorrichtung (1, 2) aufweist.

4. Anordnung nach Anspruch 1 oder 2, **dadurch gekennzeichnet, dass** die Transfereinheit (39) zur frontalen gegenüberliegenden Aufnahme von mindestens zwei Austragvorrichtungen (1, 2) ausgebildet ist, wobei die Transfereinheit Paare von durchgehenden Verbindungskanälen (43, 44) zwischen den Auslässen (17, 18; 41, 42) der Austragvorrichtungen (1, 2) aufweist.

5. Anordnung nach Anspruch 1, **dadurch gekennzeichnet, dass** die Transfereinheit (31) Verbindungskanäle (38) von den Auslässen (17, 18; 41, 42) der Austragvorrichtungen (1, 2) zu einem gemeinsamen Anschluss (32) mit mindestens einem Auslass (33, 34) für einen Mischer (25) oder ein Zubehör aufweist.

6. Anordnung nach einem der Ansprüche 1 bis 5, **dadurch gekennzeichnet, dass** mindestens ein Vorratsbehälter (5, 6) eine Mischeinrichtung (4) aufweist.

7. Anordnung nach einem der Ansprüche 1 bis 6, **dadurch gekennzeichnet, dass** die Austragvorrichtungen Doppelspritzen (1, 2) mit Doppelstösseln (9, 10) sind.

8. Anordnung nach einem der Ansprüche 1 bis 6, **dadurch gekennzeichnet, dass** die Austragvorrichtungen Doppelkartuschen mit Austragkolben sind.

9. Verfahren zum Transfer, Mischen und Austragen von Komponenten mit einer Anordnung gemäss dem Ansprüchen 1 bis 8, **dadurch gekennzeichnet, dass** die Austragvorrichtungen (1, 2) an die Transfereinheit angekuppelt werden, die eine Austragvorrichtung (2) für die flüssigen Komponenten (21, 23) betätigt wird, um diese Komponenten in die anderen Komponenten (22, 24) einzubringen und die Komponenten in der anderen Austragvorrichtung (1) gemischt werden und das Gemisch (28, 29) ausgetragen wird.

## Claims

1. Arrangement for transferring, mixing, and dispensing components that comprises at least two dispensing devices (1, 2), at least one (2) of the dispensing devices containing at least one liquid component (21, 23) and the arrangement comprising a transfer unit (15, 31, 39) for the connection of the dispensing devices that comprises connecting channels (16, 38; 43, 44) between two outlets (17, 18; 41, 42) of the components (21, 23; 22, 24) of the adjacent or opposite dispensing devices (1, 2), the at least two dispensing devices (1, 2) each comprising at least two storage containers (5 - 8), **characterised in that** the storage containers are connected to each other by a flange (19, 20) and that the transfer unit (15, 31, 39) has locking members (36) engaging behind corresponding locking members (37) on the flanges (19, 20) of the dispensing devices (1, 2).

2. Arrangement according to claim 1, **characterised in that** the connecting channels (16) of the removably attachable transfer unit (15, 39) are configured for transferring the liquid components (21, 23) from one (2) of the dispensing devices to another dispensing device (1) that is arranged in parallel thereto.

3. Arrangement according to claim 2, **characterised in that** the transfer unit (15) comprises connecting channels (16) between one outlet (17, 18; 41, 42) respectively of one storage container (5, 7; 6, 8) of the one or the other dispensing device (1, 2).

4. Arrangement according to claim 1 or 2, **characterised in that** the transfer unit (39) is adapted for receiving at least two frontally opposed dispensing devices (1, 2), the transfer unit having pairs of through-going connecting channels (43, 44) between the outlets (17, 18; 41, 42) of the dispensing devices (1, 2).

5. Arrangement according to claim 1, **characterised in that** the transfer unit (31) comprises connecting channels (38) from the outlets (17, 18; 41, 42) of the dispensing devices (1, 2) to a common coupling (32) having at least one outlet (33, 34) for a mixer (25) or an accessory.

6. Arrangement according to one of claims 1 to 5, **characterised in that** at least one of the storage containers (5, 6) comprises a mixing arrangement (4).

7. Arrangement according to one of claims 1 to 6, **characterised in that** the dispensing devices are double syringes (1, 2) with double thrust rods (9, 10).

8. Arrangement according to one of claims 1 to 6, **characterised in that** the dispensing devices are double cartridges with dispensing pistons.

9. Method for transferring, mixing, and dispensing components by means of an arrangement according to claims 1 to 8, **characterised in that** the dispensing devices (1, 2) are coupled to the transfer unit, the one dispensing device (2) for the liquid components (21, 23) is actuated for introducing these components into the other components (22, 24), and the components are mixed in the other dispensing device (1) and the mixture (28, 29) is dispensed.

## Revendications

1. Agencement pour transférer, mélanger et distribuer des composants, comprenant au moins deux dispositifs de distribution (1, 2), au moins un (2) des dispositifs de distribution contenant au moins un composant liquide (21, 23), et l'agencement comprenant une unité de transfert (15, 31, 39) permettant le raccordement des dispositifs de distribution, laquelle présente des canaux de liaison (16, 38; 43, 44) entre deux orifices de sortie (17, 18; 41, 42) des composants (21, 23; 22, 24) des dispositifs de distribution adjacents ou opposés (1, 2), et les au moins deux dispositifs de distribution (1, 2) comprenant chaque fois au moins deux réservoirs (5 - 8), **caractérisé en ce que** les réservoirs sont reliés entre eux par un flasque (19, 20), et que l'unité de transfert (15, 31, 39) comprend des éléments de verrouillage (36) qui s'engagent derrière des éléments de verrouillage (37) correspondants sur les flasques (19, 20) des dispositifs de distribution (1, 2).

2. Agencement selon la revendication 1, **caractérisé en ce que** les canaux de liaison (16) de l'unité de transfert (15, 39) amovible sont adaptés à transférer les composants liquides (21, 23) de l'un (2) des dispositifs de distribution dans un autre dispositif de distribution (1) agencé parallèlement à celui-là.

3. Agencement selon la revendication 2, **caractérisé en ce que** l'unité de transfert (15) comprend des canaux de liaison (16) entre un orifice de sortie (17, 18; 41, 42) d'un réservoir (5, 7; 6, 8) de l'un et de l'autre dispositif de distribution (1, 2), respectivement.

4. Agencement selon la revendication 1 ou 2, **caractérisé en ce que** l'unité de transfert (39) est adaptée à recevoir au moins deux dispositifs de distribution (1, 2) frontalement opposés, l'unité de transfert ayant des paires de canaux de liaison (43, 44) traversants entre les orifices de sortie (17, 18; 41, 42) des dispositifs de distribution (1, 2).

5. Agencement selon la revendication 1, **caractérisé en ce que** l'unité de transfert (31) comprend des canaux de liaison (38) depuis les orifices de sortie (17, 18; 41, 42) des dispositifs de distribution (1, 2) vers un raccordement commun (32) ayant au moins un orifice de sortie (33, 34) pour un mélangeur (25) ou un accessoire.

6. Agencement selon l'une des revendications 1 à 5, **caractérisé en ce qu'**un au moins des réservoirs (5, 6) comporte un agencement de mélange (4).

7. Agencement selon l'une des revendications 1 à 6, **caractérisé en ce que** les dispositifs de distribution sont des seringues doubles (1, 2) avec des poussoirs doubles (9, 10).

8. Agencement selon l'une des revendications 1 à 6, **caractérisé en ce que** les dispositifs de distribution sont des cartouches doubles avec des pistons de distribution.

9. Procédé pour transférer, mélanger et distribuer des composants au moyen d'un agencement selon les revendications 1 à 8, **caractérisé en ce que** les dispositifs de distribution (1, 2) sont accouplés à l'unité de transfert, l'un (2) des dispositifs de distribution pour les composants liquides (21, 23) est actionné pour introduire ces composants dans les autres composants (22, 24), et les composants sont mélangés dans l'autre dispositif de distribution (1) et le mélange (28, 29) est distribué.
